# EUROPÄISCHE PATENTSCHRIFT

(11) **EP 0 925 783 B1**
(45) Veröffentlichungstag und Bekanntmachung des Hinweises auf die Patenterteilung: **31.03.2004**
(21) Anmeldenummer: 98811237.1
(22) Anmeldetag: 16.12.1998
(51) Int. Cl.: A61K 7/48, A61K 7/40

(54) **Hautschutzzubereitung**
Skin protection preparation
Préparation pour la protection de la peau

(30) Priorität: 16.12.1997 CH 288497
(43) Veröffentlichungstag der Anmeldung: 30.06.1999
(73) Patentinhaber: Spirig Pharma AG, CH-4622 Egerkingen (CH)
(72) Erfinder: Birrenbach, Gerd, Dr., 4616 Kappel (CH); Gabard, Bernard, Dr., 4622 Egerkingen (CH)
(74) Vertreter: Braun, André

(56) Entgegenhaltungen:
- WO-A-96/19183
- FR-A- 1 338 860
- GB-A- 903 407
- US-A- 4 551 330

## Beschreibung

Die vorliegende Erfindung betrifft eine Hautschutzzubereitung für die vorbeugende Verhütung von Hautschäden. Hautschäden als Folge von direkten äusseren Einflüssen von toxischen Substanzen treten sehr oft als Hautekzeme und Irritationen auf. Insbesondere sind berufsbedingte Hautekzeme häufig zu beobachten. Solche Hauterkrankungen können bei Anwendung von geeigneten vorsorglichen Schutzmassnahmen häufig verhindert werden. Das Tragen von Handschuhen bietet zwar in der Regel guten Schutz, jedoch ist diese Massnahme nicht immer angebracht oder optimal. In vielen Fällen wäre es besser, wenn man als Verhütungsmassnahme oder zur Prophylaxe eine geeignete Hautschutzzubereitung bzw. ein geeignetes Hautschutzpräparat zur Verfügung hätte. In diesem Sinne betrifft die vorliegende Erfindung ein Hautschutzpräparat zum Aufbringen auf die Haut, um als Präventivmassnahme die degenerative Einwirkung von exogenen, Hautschäden verursachenden, toxische Stoffen auszuschalten oder zumindest zu vermindern.

Aus der Literatur, Seifen Öle Fette Wachse, 14, Seiten 1041-1044 (1995), ist bekannt, dass Aluminium(III)-Ionen in Lösung in verschiedenen Formen bzw. Komplexen vorkommt, deren Bildung und Stabilität vom im Herstellungsverfahren verwendeten pH-Wert, der Temperatur und der Konzentration abhängig ist. Diese Formen können als anorganische Polymere betrachtet werden, wobei es Oligomere unterschiedlicher Grösse gibt. Beispielsweise ist ein relativ grosses und stabiles Polymer-Ion bekannt, welches der Formel Al₁₃O₄(OH)₂₄⁷⁺ entspricht und als 13-mer bezeichnet werden kann.

FR 1 338 860 beschreibt eine Zusammensetzung bestehend aus:
(i) wasserlöslichem Polyvinylalkohol, Hydrolysierungsgrad >60%,
(ii) Weichmacher,
(iii) "adhesives" (z.B. Gelatine),
(iv) "mouillants" (z.B. PEG, Ester höherer Alkohole),
(v) Konservierungsmittel (Verbindungen der Gruppen II, III, IV, und VIII des Periodischen Systems, z.B. Aluminiumchlorid, Aluminium-hydroxychloride).

US 4 551 330 beschreibt eine Öl-in-Wasser (o/w) Emulsion, welche sich bei der Anwendung in eine Wasser-in-Öl Emulsion umwandelt (".. the compositions comprise oil-in-water emulsions that are adapted to invert to water-in-oil emulsions at the ... skin surface ....") enthaltend Wasser sowie
(i) ein "unctuous oleaginous material" (in Wasser unlösliche Wachse, Fette, Öle, Silikone, ungesättigte Ester gesättigter Säuren),
(ii) Emulgatoren (z.B. PEG, Glycerylstearat, (C₁₂-C₁₈) Fettalkohole und Ester derselben),
(iii) in Wasser dispergierbare Metallsalze von Aluminium, Cerium, Eisen, Zirkonium, sowie Aluminium/Zirkonium,
(iv) gegebenenfalls fragrances, coloring agents, preservatives, thickeners,
(v) gegebenenfalls therapeutische Substanzen, wobei der p_{H}-Wert 1.5 - 7 beträgt.

GB 903 407 betrifft "a stable antibacterial antiperspirant", d.i. eine stabile Zusammensetzung mit der antibakteriellen Wirkung des Antibiotikums Neomycin. Der Wirkungsmechanismus besteht darin, dass Neomycin die schweissabbauenden Bakterien bekämpft und dadurch schlechten Körpergeruch verhindert. In diesem Sinne wird in D3 ein antibakteriell wirkendes Antitranspirans geschützt, als Kombination der beiden Komponenten Neomycin und Aluminiumhydroxychlorid.

In analoger Weise liegt Aluminiumchlorohydrat als anorganisches Polymer mit hoher, unterschiedlicher Molekulargewichtsverteilung vor. Unter spezifischen Herstellungs-bedingungen werden Aluminiumchlorohydrat-Verbindungen erhalten, welche ein Molekulargewicht von mittlerer Grösse und enger Verteilung aufweisen, und als aktivierte Aluminiumchlorohydrate (activated, enhanced efficacy actives) bezeichnet werden. Diese aktivierten Typen zeigen im Vergleich zu den regulären Typen eine verbesserte Wirkung bezüglich der Schweisshemmung. Beispiele für solche aktivierten Aluminiumchlorohydrate sind die Markenprodukte REACH 101, REACH 103, REACH 501 oder REACH 301, wie solche von der Firma Reheis Inc., NJ 07922, USA, hergestellt und vertrieben werden. Solche Verbindungen sind beispielsweise im US 3,904,741 oder in der EP 0 183 171 beschrieben. Im US 3,904,741 wird erwähnt, dass diese Verbindungen eine gute Löslichkeit in Alkoholen und Polyalkoholverbindungen aufweisen.

Es wurde nun gefunden, dass die erwähnten aktivierten Aluminiumchlorohydrate sich insbesondere für die Herstellung von Hautschutzzubereitungen für die vorbeugende Verhütung von Hautschäden eignen, wenn diese Zubereitungen gleichzeitig ein Feuchthaltemittel sowie ein Lipid und/oder einen Fettsäureester enthalten. Aluminiumsalze ziehen in der Regel auf der Haut auf und reichern sich speziell in den Hautfolikeln an. Es entsteht dabei ein sehr trockenes stumpfes Hautgefühl. Das Feuchthaltemittel in Kombination mit dem Lipid und/oder Fettsäureester verhindert dieses Gefühl oder vermindert es zumindest in erheblichem Masse. Dies mag mit der guten Kompatibilität des aktivierten Aluminiumchlorohydrats mit dem Feuchthaltemittel und dem Lipid und/oder Fettsäureester zusammenhängen. Ausserdem wird durch das Feuchthaltemittel die Hautfeuchtigkeit erhöht bzw. eine Austrocknung der Haut verhindert. Die erfindungsgemässe Verwendung von aktivierten Aluminiumchlorohydraten zur vorbeugenden Verhütung von Hautschäden ist noch nie beschrieben worden und ist überraschend.

Die vorliegende Erfindung ist in den Ansprüchen formuliert. Insbesondere betrifft die vorliegende Erfindung eine Hautschutzzubereitung für die vorbeugende Verhütung von Hautschäden, welche dadurch gekennzeichnet ist, dass diese Hautschutzzubereitung mindestens
(a) ein in Wasser, Wasser/Alkoholgemischen oder in Alkohol lösliches aktiviertes Aluminiumchlorohydrat;
(b) ein Feuchthaltemittel, ausgewählt aus der Gruppe enthaltend Glycerin, Sorbitol, Pentandiol, Pentylene Glycol, Hyaluronsäure und deren Alkalimetallsalze, L-Pyroglutaminsäure (C₅H₇NO₃) und deren Natriumsalz, Aloe Vera, Natriumlactat und Harnstoff;
(c) ein Lipid und/oder einen Fettsäureester und
(d) gegebenenfalls weitere in der Kosmetik oder Dermatologie zugelassene übliche Zusatzstoffe enthält.

Bevorzugt sind basische Aluminiumchloride bzw. aktivierte Aluminiumchlorohydrate, wie solche im US-Patent 3,904,741 oder im EP 0 183 171 beschrieben sind. Solche basische Aluminiumchloride sind, wie bereits erwähnt, unter dem Markennamen REACH® im Handel erhältlich. Bevorzugt sind basische Aluminiumchloride der Formel

[Al₂(OH)₍₆₋ₓ₎ . xCl]ₙ (I),

worin 0<x<6 und in der Regel keine ganze Zahl ist bzw. zu sein braucht und n anzeigt, dass die Verbindung als Polymer vorliegt, wobei die bezüglich ihres Molekulargewichts unterschiedlichen Typen mittels Chromatographie (size exclusion, HPLC) charakterisiert werden können. Bevorzugt sind Aluminiumsesquichlorohydrate und Aluminiumchlorohydrate der Formel (I), worin das Verhältnis von Aluminium zu Chlorid (Al³⁺ : Cl⁻) zwischen 1.5:1 bis 2.1 liegt. Bevorzugt sind basischen Aluminiumchloride bzw. aktivierte Aluminiumchlorohydrate der durchschnittlichen Formel [Al₂(OH)₅Cl . 2.5 H₂O]ₙ.

Das basische Aluminiumchlorid verwendet man in einer Konzentration von etwa 0.5-30 Gew.-%, vorzugsweise 2.5 -10 Gew.-% und insbesondere etwa 4-6 Gew.-%, bezogen auf das Gesamtgewicht der Zubereitung.

Das Feuchthaltemittel verwendet man in einer Konzentration von etwa 0.5-20 Gew.-%, vorzugsweise 0.5-15 Gew.-% und vorzugsweise 2-8 Gew.-%, bezogen auf das Gesamtgewicht der Zubereitung.

Das Lipid und/oder den Fettsäureester verwendet man in einer Konzentration von etwa 1.0-80 Gew.-%, vorzugsweise 1-50 Gew.-% und insbesondere etwa 1-20 Gew.-%, bezogen auf das Gesamtgewicht der Zubereitung, wobei diese Bereiche für ein einzelnes Lipid (falls nur ein Lipid verwendet wird) oder die Summe der verwendeten Lipide gilt.

Neben Aluminiumchlorohydraten oder anstelle von Aluminiumchlorohydraten können auch Aluminium-Zirkonium-Chlorohydrate verwendet werden. Im Sinne der vorliegenden Erfindung fallen Aluminium-Zirkonium-Chlorohydrate unter den Begriff Aluminiumchlorohydrate. Diese Verbindungen werden in analogen Konzentrationen verwendet. Ebenso beinhaltet der Begriff "Aluminiumchlorohydrate", analog zu den Aluminium-Zirkonium-Chlorohydraten, solche an sich bekannte Verbindungen, worin das Zirkoniumion durch ein anderes mögliches Metallion ersetzt ist.

Als Feuchthaltemittel und Moisturizers kommen die an sich bekannten in der kosmetischen Industrie angewandten Stoffe zum Einsatz. Solche sind zum Beispiel in H.P. Fiedler, Lexikon der Hilfsstoffe, Editio Cantor Verlag, Aulendorf, 1996, Seiten 636 und 1020 beschrieben. Bevorzugt sind Feuchthaltemittel oder Gemische von Feuchthaltemitteln, welche die Hydratation der Haut von etwa 100 DPM Einheiten auf etwa 300-600 DPM Einheiten erhöhen. Beispiel für geeignete Feuchthaltemittel sind Polyhydroxyverbindungen wie Glycerin, Sorbitol, Pentandiol, Pentylene Glycol, Hyaluronsäure und deren Alkalimetallsalze, vorzugsweise deren Natriumsalz, Natrium PCA, d.i. L-Pyroglutaminsäure (C₅H₇NO₃) und deren Natriumsalz, Aloe Vera, Natriumlactat, Harnstoff und andere kosmetisch verwendbare an sich bekannte Feuchthaltemittel, wie solche beispielsweise in oben angegebener Literaturstelle beschrieben sind. Bevorzugte Feuchthaltemittel sind Harnstoff, Sorbitol und Glycerin, und insbesondere Glycerin.

Lipide sind beispielsweise Pflanzenöle oder Pflanzenfette wie solche aus Sonnenblumen, Mandeln, Disteln, Soyabohnen, Sheabutter, Weizenkeimen, Oliven, Erdnüssen, Raps oder JoJoba gewonnen werden. Lipide sind im weiteren hydrierte pflanzliche Glyceride, hydrierte oder partiell hydrierte (C₁₄-C₂₀)-Fettsäureglyceride, Fettsäureester wie Isopropylmyristate; Glycerinmonostearat, (C₁₂-C₁₅)-Alkyl-benzoate, 13-Docosensäure-9-octadecenylester (Oleyl-erucate), Cetearyl-isonanoat, Octylpalmitat, Cetylpalmitat, Isooctylstearat, Butyladipat, Myristyllactat, Stearylheptanoat, Isostearylneopentanoat, 2-Octyldodecanol. Solche Lipide sind ausführlich in dem oben angegebenen Lexikon der Hilfsstoffe beschrieben.

Die erfindungsgemässen Hautschutzzubereitungen können in irgend einer kosmetisch akzeptablen, topisch anwendbaren Form vorliegen bzw. zur Anwendung gelangen, beispielsweise als Öl-in-Wasser-Emulsionen (O/W-Emulsionen), Wasser-in-Öl-Emulsionen (W/O-Emulsion), wässrige oder wässrig/alkoholische Lösungen, als Lotios, als Gele, Schäume, Salben oder Pasten oder entsprechende Mischformen, vorzugsweise als Wasser-in-Öl-Emulsionen, Öl-in-Wasser-Emulsionen, als wässrig/alkoholische Lösung oder als Salbe. Als Anwendungsform ist insbesondere die Öl-in-Wasser-Emulsion bevorzugt, wobei diese zu einer Lotio, einem Gel oder einer Salbe verarbeitet wird.

In der Kosmetik zugelassene Zusatzstoffe sind beispielsweise Emulgatoren, Tenside, Paraffinkohlenwasserstoffe, Konsistenzgeber, Gelbildner, Konservierungsmittel, Parfümierungsmittel (Parfüme), Silikone, Rückfetter, Treibgase, Füllstoffe, Farbstoffe und/oder Lösungsmittel. Wasser kann jeweils ebenfalls in den Zubereitungen enthalten sein.

Emulgatoren sind vorzugsweise nicht-ionogene Verbindungen wie beispielsweise Polyäthylenglycoläther von Cetylacohol oder von Stearylalcohol wie Polyäthylenglycol-20-cetyläther oder Polyäthylenglycol-10-stearyläther; Polyäthylenglycolstearate wie Polyäthylenglycol-40-stearat; Polyäthylenglycol-Sorbitan-Fettsäureester, wie Polyäthylenglycol-2-sorbitan-monolaurat; Polyäthylenglycol-Glycerin-Fettsäureester wie Polyäthylenglycolglycerin-monoisostearat; Polyäthylenglycol-60-Almond glycerides; Polyäthylenglycol-60-Corn glycerides; Polyäthylenglycol-45-Palmkernöl-glyceride; Methylglucosidester wie Methylglucosid-stearate; oder andere gemäss der CTFA Liste kosmetisch verwendbare Emulgatoren. Solche Emulgatoren sind ausführlich in der oben angegebenen Literatur, H.P. Fiedler, Lexikon der Hilfsstoffe, beschrieben. Emulgatoren verwendet man in einer Konzentration von etwa 0.1-30 Gew.-%, vorzugsweise 0.1-10 Gew.-% und insbesondere etwa 1-5. Gew.-%, bezogen auf das Gesamtgewicht der Zubereitung. Emulgatoren verwendet man in Öl-in-Wasser-Emulsionen, in Wasser-in-Öl-Emulsionen, in wässrigen oder wässrig/alkoholischen Lösungen, in Lotios, in Gelen, in Schäumen, in Salben oder in Pasten, vorzugsweise in Wasser-in-Öl-Emulsionen und Öl-in-Wasser-Emulsionen. Paraffinkohlenwasserstoffe sind beispielsweise Vaseline, Paraffinöle, Paraffin Wachse.

Konsistenzgeber sind vorzugsweise Wachse wie beispielsweise hydrierte pflanzliche Glyceride, hydrierte oder partiell hydrierte (C₁₄-C₂₀)-Fettsäureglyceride, Cetylpalmitat, Glycerinmonostearat, welche auch als Lipide und Fettsäureester verwendbar sind und auch als Konsistenzgeber wirken, sowie Cetylalcohol, Stearylalcohol, Behenylalcohol. Solche Konsistenzgeber sind ausführlich in dem oben angegebenen Lexikon der Hilfsstoffe beschrieben. Konsistenzgeber verwendet man in einer Konzentration von etwa 0.1-30 Gew.-%, vorzugsweise 5-20 Gew.-% und insbesondere etwa 5-10 Gew.-%, bezogen auf das Gesamtgewicht der Zubereitung. Konsistenzgeber verwendet man in Öl-in-Wasser-Emulsionen, in Wasser-in-Öl-Emulsionen, in wässrigen oder wässrig/alkoholischen Lösungen, in Lotios, Gelen, Schäumen, Salben oder in Pasten, vorzugsweise in Wasser-in-Öl-Emulsionen und Öl-in-Wasser-Emulsionen.

Gelbildner sind beispielsweise die an sich als Gelbildner bekannten verschiedenen Polymere der Acrylsäure (Carbomer) und deren Salze, Xanthan Gum, Alginic acid, Aluminiumsilikate, z.B. Natrium-Magnesium-Silikate, Cellulosederivate wie Natrium-Carboxymethylcellulose, Methylcellulose, Hydroxypropylcellulose, Hydroxyäthylcellulose, Hydroxypropylmethylcellulose und verwandte Verbindungen, Polyvinylalkohol, Polyvinylpyrrolidon, Polyäthylenglykole, Polyvinylacetat/Methylacrylat/Decadien-Copolymer, sowie weitere an sich bekannte Gelbildner, welche in der Kosmetikindustrie verwendet werden.

Konservierungsmittel sind an sich bekannt und entsprechen den international anerkannten Verbindungen wie solche beispielsweise in der Bundesrepublik Deutschland in der Verordnung über kosmetische Mittel (Kosmetik Verordnung) vom 26.6.1985 und der dazugehörigen 25. Anderungsverordnung vom 23.12.1996 gegeben sind. Bevorzugt sind Triclosan, Phenoxyäthanol, Phenyläthylalcohol, Hexamidin-isethionat, Benzylalkohol, Chlorhexidin-dihydrochlorid. Solche Konservierungsmittel verwendet man in einer Konzentration von bis zu 10 Gew.-%, vorzugsweise 0.1-5 Gew.-% und insbesondere etwa 0.1-1 Gew.-%, bezogen auf das Gesamtgewicht der Zubereitung.

Parfümierungsmittel (Parfüme) sind beispielsweise natürliche ätherische Öle, welche im Handel z.B. mit der Handelsbezeichnung Pluressence, Pulvessence oder Nonox erhältlich sind, sowie auch synthetische Geruchskorrigentien.

Silikone sind beispielsweise Cyclomethicone, Dimethicone, Phenyldimethicone, Polysiloxan-Polyalkylenoxid-Copolymere sowie weitere an sich bekannte in der Kosmetikindustrie verwendete Polymere. Silikone wirken wasserabstossend, sowie auch als Gleitmittel und Entschäumer. Silikone verwendet man in einer Konzentration von etwa 0.1-20 Gew.-%, vorzugsweise 0.2-10 Gew.-% und insbesondere etwa 0.2-0.8 Gew.-%, bezogen auf das Gesamtgewicht der Zubereitung. Silkone verwendet man in Öl-in-Wasser-Emulsionen, in Wasser-in-Öl-Emulsionen, in wässrigen oder wässrig/alkoholischen Lösungen, in Lotios, in Gelen, in Schäumen, in Salben oder in Pasten, vorzugsweise in Wasser-in-Öl-Emulsionen und Öl-in-Wasser-Emulsionen.

Rückfetter verwendet man vorzugsweise in wässrig/alkoholischen Lösungen. Rückfetter wirken der die Haut entfettenden Alkohole entgegen. Rückfetter sind beispielsweise Isostearyllactat, Glyceryl-laurat, Polyäthylenglycol-75-Lanolin, Polyäthylenglycol-7-glyceryl-cocoat, Polyäthylenglycol-15-castor oil. Verbindungen, welche als Komponente (c) aufgeführt sind, haben teilweise auch eine rückfettende Wirkung. Solche Rückfetter sind ausführlich in A. Domsch, Die kosmetischen Präparate, Band II, Verlag für chem. Industrie, H. Ziolkowsky KG, Augsburg, 1992, Seiten 203ff beschrieben. Rückfetter verwendet man in einer Konzentration von bis zu 20 Gew.-%, vorzugsweise bis zu 10 Gew.-% und insbesondere 0.1-5 Gew.-%, bezogen auf das Gesamtgewicht der Zubereitung. Rückfetter verwendet man in Öl-in-Wasser-Emulsionen, in Wasser-in-Öl-Emulsionen, in wässrigen oder wässrig/alkoholischen Lösungen, in Lotios, in Gelen, in Schäumen, in Salben oder in Pasten, vorzugsweise in wässrigalkoholischen Lösungen und in Öl-in-Wasser-Emulsionen.

Treibgase sind beispielsweise komprimierte Luft, Kohlendioxid, Propan/Butan-Gemische, Dimethyläther sowie weitere an sich bekannte in der Kosmetikindustrie verwendete Treibgase. Treibgase verwendet man in an sich bekannten Konzentrationen, insbesondere in Sprays.

Füllstoffe sind beispielsweise Titanoxide wie Titandioxid, Zinkoxid, Maisstärke, Stärkephosphate und weitere an sich bekannte in der Kosmetikindustrie verwendete Füllstoffe. Solche Füllstoffe sind in dem oben angegebenen Lexikon der Hilfsstoffe beschrieben. Füllstoffe verwendet man in einer Konzentration von bis zu 90 Gew.-%, vorzugsweise bis zu 50 Gew.-%, bezogen auf das Gesamtgewicht der Zubereitung. Füllstoffe verwendet man in Öl-in-Wasser-Emulsionen, in Wasser-in-Öl-Emulsionen, in wässrigen oder wässrig/alkoholischen Lösungen, in Lotios, in Gelen, in Schäumen, in Salben oder in Pasten, vorzugsweise in Wasser-in-Öl-Emulsionen und Öl-in-Wasser-Emulsionen.

Lösungsmittel sind beispielsweise Wasser, Äthanol, Isopropylalcohol, Propylenglycol sowie weitere an sich bekannte in der Kosmetikindustrie verwendete Lösungsmittel. Lösungsmittel verwendet man in an sich bekannten Konzentrationen.

Das Gewichtsverhältnis der drei Komponenten zueinander, nämlich Komponente a) : Komponente b) : Komponente c) ist im Bereich von 0.25-4 : 0.25-4 : 0.75-12, vorzugsweise 0.5-2 : 0.5-2 : 1.5-6, besonders bevorzugt 1 : 1 : 3.

Eine bevorzugte Formulierung enthält beispielsweise: etwa 5 Gew.-% aktiviertes Aluminiumchlorohydrat (vorzugsweise REACH 301 oder REACH 501) als Komponente (a); etwa 5 Gew.-% Glycerin als Komponente (b); und etwa 8 Gew.-% Paraffinöl, etwa 4 Gew.-% Octylpalmitat und etwa 4 Gew.-% JoJobaöl als Komponente (c); etwa 1/2 Gew.-% Dimethicon; gegebenenfalls Wasser (ca. 63 Gew.%) sowie weitere Additive, welche für die Herstellung einer bestimmten Gebrauchsform nötig sind. Die folgenden Beispiele erläutern die Erfindung.

### Beispiel 1 (Crème als O/W-Emulsion)

a) 62.9 Teile destilliertes Wasser (aqua purificata), 5.0 Teile Glycerin (Feuchthaltemittel), 5.0 Teile Aluminiumchlorhydrat (entsprechend der chemischen Formel [Al₂(OH)₅]ₙ . nCl), sowie 0.1 Teile Hexamidin-isethionat (Konservierungsmittel), werden in einem Mischer zusammen bei einer Temperatur von 80°C gelöst.
b) 8.0 Teile Paraffinöl, 4.0 Teile Octylpalmitat, 4.0 Teile Jojobaöl, 7.5 Teile Behenylalkohol, 1.5 Teile Polyäthylenglykol(PEG)-10-cetyläther (Ceteth-10) und 1.5 Teile Polyäthylenglycol-20-stearyläther (Stearath-20) werden in einem Mischer bei 80°C geschmolzen und unter Rühren sowie Homogenisieren zu der unter a) erhaltenen Zusammensetzung zugegeben. Die erhaltene Mischung wird nun unter weiterem Rühren auf 60°C abkühlen gelassen. Anschliessend wird auf 25°C abkühlen gelassen und während zehn bis zwanzig Minuten weiter gerührt. Dabei werden 0.5 Teile Phenoxyäthanol (Konservierungsmittel) zugegeben, wobei eine Creme homogener Konsistenz erhalten wird. Die Creme kann nun in Tuben abgefüllt werden.

### Beispiel 2 (Crème als W/O -Emulsion)

a) 6.0 Teile Octylpalmitat, 4.0 Teile Shea-Butter, 2.0 Teile Dimethicone, 5.0 Teile Methoxy-PEG-22-/Dodecyl-Glycol-Copolymer, 2.0 Teile PEG-45/Dodecyl-Glycol-Copolymer und 4.0 Teile Hydroxyoctacosanyl-hydroxystearat werden in einem Mischer bei 80°C geschmolzen.
b) 54.4 Teile destilliertes Wasser, 2.0 Teile Natrium PCA (Natrium-L-Pyroglutaminsäure), 20.0 Teile Aluminiumchlorhydrat (REACH® 501), sowie 0.1 Teile Hexamidin-isethionat (Konservierungsmittel), werden in einem Mischer zusammen bei einer Temperatur von 80°C gelöst und unter Rühren sowie Homogenisieren zu der unter a) erhaltenen Schmelze zugegeben. Die erhaltene Mischung wird nun unter weiterem Rühren auf 50°C abkühlen gelassen. Anschliessend wird auf 25°C abkühlen gelassen und während zehn bis zwanzig Minuten weiter gerührt. Dabei werden 0.5 Teile Phenoxyäthanol zugegeben, wobei eine Creme homogener Konsistenz erhalten wird. Die Creme kann nun in Tuben abgefüllt werden.

### Beispiel 3 (Lotio als O/W-Emulsion)

a) 66.0 Teile destilliertes Wasser (aqua purificata), 5.0 Teile Glycerin (Feuchthaltemittel), 10.0 Aluminiumchlorhydrat (REACH® 501), werden in einem Mischer zusammen bei einer Temperatur von 80°C gelöst.
b) 8.0 Teile Octyldodecanol, 8.0 Teile partiell hydrierte (C₁₄-C₂₀)-Fettsäureglyceride und 3.0 Teile Methylglucosidstearate werden in einem Mischer bei 80°C geschmolzen und unter Rühren sowie Homogenisieren der unter a) erhaltenen Zusammensetzung zugegeben. Die erhaltene Mischung wird nun unter weiterem Rühren auf 60°C abkühlen gelassen. Anschliessend wird auf 25°C abkühlen gelassen und während zehn bis zwanzig. Minuten weiter gerührt. Die erhaltene Lotio kann nun abgefüllt werden.

### Beispiel 4 (Lotio als O/W-Emulsion)

a) 67.0 Teile destilliertes Wasser, 4.0 Teile Sorbitol, 10.0 Aluminiumchlorhydrat (REACH® 501), werden in einem Mischer zusammen bei einer Temperatur von 80°C gelöst.
b) 8.0 Teile Octyldodecanol, 8.0 Teile Glyceryl-dioleat und 3.0 Teile Methylglucosid-stearat werden in einem Mischer bei 80°C geschmolzen und unter Rühren sowie Homogenisieren zu der unter a) erhaltenen Zusammensetzung zugegeben wobei die Mischung während 10 Minuten homogenisiert wird. Die erhaltene Mischung wird nun unter weiterem Rühren auf Raumtemperatur abkühlen gelassen. Die erhaltene Lotio kann nun abgefüllt werden.

### Beispiel 5 (wässrig/alkoholische Lösung)

a) 52.0 Teile destilliertes Wasser (aqua purificata), 15.0 Teile Äthanol (96%), 5.0 Teile Glycerin und 20.0 Teile Aluminiumchlorhydrat (REACH® 501), werden in einem Mischer zusammen bei Raumtemperatur gelöst.
b) 8.0 Teile Polyäthylenglycol-15-Ricinusöl werden anschliessend unter Rühren zu der unter a) erhaltenen Zusammensetzung zugegeben, bis eine homogenen Lösung erhalten wird. Die erhaltene Lösung kann nun in Flaschen abgefüllt werden.

### Beispiel 6 (wässrig/alkoholische Lösung)

a) 52.0 Teile destilliertes Wasser, 15.0 Teile Äthanol (96%), 5.0 Teile Pentylenglycol und 20.0 Teile Aluminiumchlorhydrat (REACH® 501), werden in einem Mischer zusammen bei Raumtemperatur gelöst.
b) 8.0 Teile Polyäthylenglycol-15-Castor Oil werden anschliessend unter Rühre zu der unter a) erhaltenen Zusammensetzung zugegeben, bis eine homogenen Lösung erhalten wird. Die erhaltene Lösung kann nun in Pumpspray-Flaschen abgefüllt werden.

### Beispiel 7 (Gel)

a) 8.0 Teile PEG-7-glyceryl-cocoate, 3.0 Teile Natriumlactat und 10.0 Teile Aluminiumchlorhydrat (REACH® 501), werden in einem Mischer zusammen bei einer Temperatur von 80°C gemischt.
b) 75.0 Teile destilliertes Wasser und 4.0 Teile Magnesium-Aluminum-Silikat werden in einem Mischer bei 80°C zu einer Dispersion verarbeitet. Diese Dispersion wird nun in die unter a) erhaltene Mischung eingerührt, mit einem Homogenisator während 10 Minuten homogenisiert und anschliessend gerührt bis das erhaltenen Gel auf Raumtemperatur abgekühlt ist. Das Gel kann nun in Tuben abgefüllt werden.

### Beispiel 8 (Schaum)

a) 72.0 Teile destilliertes Wasser, 10.0 Teile Harnstoff, 5.0 Aluminiumchlorhydrat (REACH® 501) sowie 0.5 Teile Benzylalkohol werden in einem Mischer zusammen bei einer Temperatur von 80°C gelöst.
b) 8.0 Teile Octylstearat und 4.0 Teile Polyäthylenglycol-20-stearyläther (Stearath-20) werden in einem Mischer bei 80°C zusammen geschmolzen, unter Rühren sowie Homogenisieren der unter a) erhaltenen Zusammensetzung zugegeben und während 10 Minuten homogenisiert. Die erhaltene Emulsion wird nun unter weiterem Rühren auf 25°C abkühlen gelassen. Die Emulsion kann nun in Acrosol-Dosen abgefüllt und mit einem Propan/Butan-Gemisch (0.5 Gew.-%, berechnet auf das Gesamtgewicht der Emulsion) begast werden.

### Beispiel 9 (Salbe)

a) 22.5 Teile Paraffinöl, 28.0 Teile Petrolatum und 15.0 Teile Ozokerite werden in einem Mischer bei 80°C geschmolzen. Anschliessend werden 0.5 Teile Aluminiumstearat der Schmelze unter Rühren bei 80°C zugesetzt und gelöst.
b) Der unter a) erhaltenen Schmelze werden nun 24.0 Teile Isopropylmyristat, 7.0 Teile Aluminiumchlorhydrat (REACH® 501), sowie 3.0 Teile Pentylenglycol bei 80°C und unter Rühren zugesetzt und mit einem Homogenisator während 10 Minuten homogenisiert. Es wird unter Rühren auf Raumtemperatur abkühlen gelassen. Die erhaltene Salbe kann in Tuben abgefüllt werden.

### Beispiel 10 (Paste)

a) 48.0 Teile aqua purificata, 10.0 Teile Glycerin, 15.0 Teile Aluminiumchlorhydrat (REACH® 501) werden in einem Mischer zusammen bei einer Temperatur von 80°C gelöst.
b) 6.0 Teile Paraffinöl, 4.0 Teile (C₁₂-C₁₅)-Alkylbenzoat, 2.0 Teile Dimethicone, 2.6 Teile Polyäthylenglycol-5-stearylstearat, 2.4 Teile Polyäthylenglycol-40-stearat, 3.0 Teile Cetylpalmitat, 2.0 Teile Glycerylstearat und 3.0 Teile Glycolpalmitat werden in einem Mischer bei einer Temperatur von 80°C geschmolzen und unter Rühren zu der unter a) erhaltenen Zusammensetzung zugegeben. Die erhaltene Mischung wird nun während 10 Minuten mit einem Homogenisator bei einer Temperatur von 80°C homogenisiert. Anschliessend werden bei derselben Temperatur 5.0 Teile Titandioxid und 5.0 Teile Distärkephosphat (distarchphosphate) zugesetzt. Die erhaltene Zusammensetzung wird nun während 15 Minuten und unter weiterem Rühren bei 80°C mit einem Mixer behandelt bis eine gleichmässige Suspension erhalten wird. Dann wird abkühlen gelassen. Die Paste kann nun in Tuben abgefüllt werden.

## Patentansprüche

1. Hautschutzzubereitung für die vorbeugende Verhütung von Hautschäden, **dadurch gekennzeichnet, dass** diese Hautschutzzubereitung mindestens
(a) ein in Wasser, Wasser/Alkoholgemischen oder in Alkohol lösliches aktiviertes Aluminiumchlorohydrat;
(b) ein Feuchthaltemittel, ausgewählt aus der Gruppe enthaltend Glycerin, Sorbitol, Pentandiol, Pentylene Glycol, Hyaluronsäure und deren Alkalimetallsalze, L-Pyroglutaminsäure (C₅H₇NO₃) und deren Natriumsalz, Aloe Vera, Natriumlactat und Harnstoff;
(c) ein Lipid und/oder einen Fettsäureester und
(d) gegebenenfalls weitere in der Kosmetik oder Dermatologie zugelassene übliche Zusatzstoffe enthält.

2. Hautschutzzubereitung nach Anspruch 1, **dadurch gekennzeichnet, dass** das aktivierte Aluminiumchlorohydrat der Formel
[Al₂(OH)₍₆₋ₓ₎ . xCl]ₙ (I)
entspricht, worin 0<x<6 und in der Regel keine ganze Zahl ist bzw. zu sein braucht und n anzeigt, dass die Verbindung als Polymer vorliegt.

3. Hautschutzzubereitung nach Anspruch 1 oder 2, **dadurch gekennzeichnet, dass** das aktivierte Aluminiumchlorohydrat ein Aluminiumsesquichlorohydrat und/oder ein Aluminiumchlorohydrat der Formel (I) darstellt, worin das Verhältnis von Aluminium zu Chlorid (Al³⁺ : Cl⁻) zwischen 1.5:1 bis 2.1 liegt.

4. Hautschutzzubereitung nach einem der Ansprüche 1 bis 3, **dadurch gekennzeichnet, dass** das aktivierte Aluminiumchlorohydrat der durchschnittlichen Formel [Al₂(OH)₅Cl . 2.5 H₂O]ₙ entspricht.

5. Hautschutzzubereitung nach einem der Ansprüche 1 bis 4, **dadurch gekennzeichnet, dass** das aktivierte Aluminiumchlorohydrat in einer Konzentration von 0.5-30 Gew.-%, vorzugsweise 2.5 -10 Gew.-% und insbesondere etwa 4-6 Gew.-%, bezogen auf das Gesamtgewicht der Zubereitung.

6. Hautschutzzubereitung nach einem der Ansprüche 1 bis 5, **dadurch gekennzeichnet, dass** das Feuchthaltemittel in einer Konzentration von 0.5-20 Gew.-%, vorzugsweise 0.5-15 Gew.-% und vorzugsweise 2-8 Gew.-%, bezogen auf das Gesamtgewicht der Zubereitung.

7. Hautschutzzubereitung nach einem der Ansprüche 1 bis 6, **dadurch gekennzeichnet, dass** das Lipid und/oder der Fettsäureester in einer Konzentration von 1.0-80 Gew.-%, vorzugsweise 1-50 Gew.-% und insbesondere 1-20 Gew.-%, bezogen auf das Gesamtgewicht der Zubereitung.

8. Hautschutzzubereitung nach einem der Ansprüche 1 bis 7, **dadurch gekennzeichnet, dass** neben Aluminiumchlorohydrat oder anstelle von Aluminiumchlorohydrat auch ein Aluminium-Zirkonium-Chlorohydrat anwesend ist.

9. Hautschutzzubereitung nach einem der Ansprüche 1 bis 8, **dadurch gekennzeichnet, dass** diese als Feuchthaltemittel eine Verbindung enthält, welche die Hydratation der Haut von etwa 100 DPM Einheiten auf 300-600 DPM Einheiten erhöht.

10. Hautschutzzubereitung nach einem der Ansprüche 1 bis 10, **dadurch gekennzeichnet, dass** diese als Feuchthaltemittel Glycerin, Sorbitol, Aloe Vera, Natriumlactat und/oder Harnstoff, vorzugsweise Glycerin, enthält.

11. Hautschutzzubereitung nach einem der Ansprüche 1 bis 10, **dadurch gekennzeichnet, dass** diese als Lipid ein Pflanzenöl und/oder Pflanzenfett wie solche aus Sonnenblumen, Mandeln, Disteln, Soyabohnen, Sheabutter, Weizenkeimen, Oliven, Erdnüssen, Raps oder JoJoba gewonnen werden, und/oder Fettsäureester, vorzugsweise Isopropylmyristate; (C₁₂-C₁₅)-Alkyl-benzoate, 13-Docosensäure-9-octadecenylester (Oleyl-erucate), Cetearyl-iso-nanoat, Octylpalmitat, Isooctylstearat, Butyladipat, Myristyllactat, Stearylheptanoat, Isostearylneopentanoat und/oder 2-Octyldodecanol enthält.

12. Hautschutzzubereitung nach einem der Ansprüche 1 bis 11, **dadurch gekennzeichnet, dass** diese in einer kosmetisch akzeptablen, topisch anwendbaren Form vorliegen, vorzugsweise als Öl-in-Wasser-Emulsion, Wasser-in-Öl-Emulsion, wässrige oder wässrig/alkoholische Lösung, als Lotio, als Gel, als Schaum, Salbe oder Paste, oder in einer entsprechenden Mischform, vorzugsweise als Wasser-in-Öl-Emulsionen, Öl-in-Wasser-Emulsion, als wässrig/alkoholische Lösung oder als Salbe, vorzugsweise als Öl-in-Wasser-Emulsion in Form einer Lotio, eines Gels oder einer Salbe.

13. Hautschutzzubereitung nach einem der Ansprüche 1 bis 12, **dadurch gekennzeichnet, dass** diese einen in der Kosmetik zugelassenen Zusatzstoff enthält ausgewählt aus der Gruppe der Emulgatoren, Tenside, Paraffinkohlenwasserstoffe, Konsistenzgeber, Gelbildner, Konservierungsmittel, Parfümierungsmittel, Silikone, Rückfetter, Treibgase, Füllstoffe, Farbstoffe und/oder Lösungsmittel.

14. Hautschutzzubereitung nach einem der Ansprüche 1 bis 13, **dadurch gekennzeichnet, dass** das Gewichtsverhältnis der Komponente a) : Komponente b) : Komponente c) im Bereich von 0.25-4:0.25-4:0.75-12, vorzugsweise 0.5-2:0.5-2:1.5-6, besonders bevorzugt 1:1:3 beträgt.

15. Hautschutzzubereitung nach einem der Ansprüche 1 bis 14, **dadurch gekennzeichnet, dass** diese etwa 5 Gew.-% aktiviertes Aluminiumchlorohydrat (vorzugsweise REACH 301 oder REACH 501) als Komponente (a); etwa 5 Gew.-% Glycerin als Komponente (b); und etwa 8 Gew.-% Paraffinöl, etwa 4 Gew.-% Octylpalmitat und etwa 4 Gew.-% JoJobaöl als Komponente (c); etwa 1/2 Gew.-% Dimethicon; gegebenenfalls Wasser (ca. 63 Gew.%) sowie weitere Additive, welche für die Herstellung einer bestimmten Gebrauchsform nötig sind, enthält.

16. Verwendung der Zubereitung nach einem der Ansprüche 1 bis 15, als Hautschutzmittel.

## Claims

1. A skin protection preparation for the prophylactic prevention of skin damages, **characterised in that** said skin protection preparation contains at least:
(a) an activated aluminium chlorohydrate, which is soluble in water, water/alcohol mixtures or alcohol;
(b) a moisturising agent, which is selected from the group comprising glycerol, sorbitol, pentandiol, pentylene glycol, hyaluronic acid and alkali metal salts thereof, L-pyroglutaminic acid (C₅H₇NO₃) and sodium salts thereof, aloe vera, sodium lactate and urea;
(c) a lipid and/or a fatty acid ester and
(d) optionally further conventional additives which are acceptable in cosmetics or dermatology.

2. A skin protection preparation according to claim 1, **characterised in that** the activated aluminium chlorohydrate corresponds to the formula
[Al₂(OH)₍₆₋ₓ₎ . XCl]ₙ (I)
wherein 0<x<6 and as a rule is not a whole number, resp. does not need to be a whole number, and wherein n indicates that the compound is present in the form of a polymer.

3. A skin protection preparation according to claim 1 or 2, **characterised in that** the activated aluminium chlorohydrate represents an aluminium sesquichlorohydrate and/or an aluminium chlorohydrate of formula (I), wherein the ratio of aluminium to chloride (Al³⁺ : Cl⁻) is in the range of from 1.5:1 to 2.1.

4. A skin protection preparation according to any one of the claims 1 to 3, **characterised in that** the activated aluminium chlorohydrate corresponds to the average formula [Al₂(OH)₅Cl . 2.5 H₂O]ₙ.

5. A skin protection preparation according to any one of the claims 1 to 4, **characterised in that** the activated aluminium chlorohydrate is present in a concentration of 0.5-30% by weight, preferably 2.5-10% by weight and especially about 4-6% by weight, with reference to the total weight of the composition.

6. A skin protection preparation according to any one of the claims 1 to 5, **characterised in that** the moisturising agent is present in a concentration of 0.5-20% by weight, preferably 0.5-15% by weight and preferably 2-8% by weight, with reference to the total weight of the composition.

7. A skin protection preparation according to any one of the claims 1 to 6, **characterised in that** the lipid and/or the fatty acid ester is present in a concentration of 1.0-80% by weight, preferably 1-50% by weight and especially 1-20% by weight, with reference to the total weight of the composition.

8. A skin protection preparation according to any one of the claims 1 to 7, **characterised in that** besides the aluminium chlorohydrate or in the place of aluminium chlorohydrate also an aluminium-zirconium-chlorohydrate is present.

9. A skin protection preparation according to any one of the claims 1 to 8, **characterised in that** said composition contains as a moisturising agent a compound which increases the hydration of the skin from about 100 DPM units to 300-600 DPM units.

10. A skin protection preparation according to any one of the claims 1 to 10, **characterised in that** said composition contains as a moisturising agent glycerol, sorbitol, aloe vera, sodium lactate and/or urea, preferably glycerol.

11. A skin protection preparation according to any one of the claims 1 to 10, **characterised in that** said composition contains as a lipid a vegetable oil and/or a vegetable fat as is obtained from sunflower, almonds, thistles, soya beans, shea butter, wheat germs, olives, peanuts, rape or jojoba, and/or fatty acid esters, preferably isopropyl myristate; (C₁₂-C₁₅)-alkyl-benzoates, 13-docosenylacid-9-octadecenylester (oleylerucate), cetearyl-iso-nanoate, octyl palmitate, isooctyl stearate, butyl adipate, myristyl lactate, stearyl heptanoate, isostearyl neopentanoate and/or 2-octyldodecanol.

12. A skin protection preparation according to any one of the claims 1 to 11, **characterised in that** said composition is present in a cosmetically acceptable topically applicable form, preferably as an oil-in-water emulsion, water-in-oil emulsion, aqueous or aqueous/alcoholic solution, as a lotio, a gel, a foam, an ointment or a paste, or in a corresponding mixed form, preferably as water-in-oil-emulsion, oil-in-water-emulsion, as an aqueous/alcoholic solution or an ointment, preferably as an oil-in-water-emulsion in the form of a lotio, a gel or an ointment.

13. A skin protection preparation according to any one of the claims 1 to 12, **characterised in that** said composition contains a cosmetically acceptable additive selected from the group comprising emulsifying agents, tensides, paraffin-hydrocarbons, consistency regulating agent, gelling agents, preservatives, perfumes, silicones, refattening agents, propellants, fillers, colouring agents and/or solvents.

14. A skin protection preparation according to any one of the claims 1 to 13, **characterised in that** the weight ratio of component a) : component b) : component c) is within the range of 0.25-4:0.25-4:0.75-12, preferably 0.5-2:0.5-2:1.5-6, especially preferred 1:1:3.

15. A skin protection preparation according to any one of the claims 1 to 14, **characterised in that** said composition contains about 5% by weight of activated aluminium chlorohydrate (preferably REACH 301 or REACH 501) as component (a); about 5% by weight glycerol as component (b); and about 8% by weight paraffin oil, about 4% by weight octyl palmitate and about 4% by weight jojoba oil as component (c); about 0.5% by weight dimethicon; optionally water (about 63% by weight) as well as further additives which are necessary to the preparation of a selected form to be used.

16. The use of a preparation according to any one of the claims 1 to 15 as a skin protection preparation.

## Revendications

1. Préparation pour la protection de la peau pour la prévention prophylactique de dommages de la peau, **caractérisée en ce que** ladite préparation pour la protection de la peau contient au moins
(a) un chlorhydrate d'aluminium activé soluble dans l'eau, les mélanges d'eau/alcool ou l'alcool ;
(b) un agent humectant choisi dans le groupe comprenant glycérine, sorbitol, pentandiol, pentylène glycol, acide hyaluronique et leurs sels de métal alcalin, acide L-pyroglutaminique (C₅H₇NO₃) et leurs sels de sodium, aloe vera, lactate de sodium et urée ;
(c) un lipide et/ou un ester d'acide gras et
(d) optionnellement d'autres additifs conventionnels qui sont acceptables dans les cosmétiques ou la dermatologie.

2. Préparation pour la protection de la peau selon la revendication 1, **caractérisée en ce que** ledit chlorhydrate d'aluminium activé correspond à la formule
[Al₂(OH)₍₆₋ₓ₎.xCl]ₙ (I)
où 0<x<6 et n'est en général pas un chiffre entier resp. ne doit pas l'être et où n indique que le composé est présent sous forme d'un polymère.

3. Préparation pour la protection de la peau selon la revendication 1 où 2, **caractérisée en ce que** ledit chlorhydrate activé représente un sesquichlorhydrate d'aluminium et/ou un chlorhydrate d'aluminium de la formule (I), où la proportion d'aluminium par rapport au chlorure (Al³⁺ : Cl⁻) est compris entre 1.5 :1 et 2.1.

4. Préparation pour la protection de la peau selon l'une quelconque des revendications 1 à 3, **caractérisée en ce que** ledit chlorhydrate d'aluminium activé correspond à la formule moyenne [Al₂(OH)₅Cl. 2.5 H₂O]ₙ.

5. Préparation pour la protection de la peau selon l'une quelconque des revendications 1 à 4, **caractérisée en ce que** ledit chlorhydrate d'aluminium activé est présent en une concentration de 0.5-30 % en poids, de préférence de 2.5-10 % en poids et particulièrement d'environ 4-6 % en poids par rapport au poids total de la préparation.

6. Préparation pour la protection de la peau selon l'une quelconque des revendications 1 à 5, **caractérisée en ce que** l'agent humectant est présent en une concentration de 0.5-20 % en poids, de préférence de 0.5-15 % en poids et particulièrement de 2-8 % en poids par rapport au poids total de la préparation.

7. Préparation pour la protection de la peau selon l'une quelconque des revendications 1 à 6, **caractérisée en ce que** le lipide et/ou l'ester d'acide gras est présent en une concentration de 1.0-80 % en poids, de préférence de 1-50 % en poids et particulièrement de 1-20 % en poids par rapport au poids total de la préparation.

8. Préparation pour la protection de la peau selon l'une quelconque des revendications 1 à 7, **caractérisée en ce qu'**en plus de chlorhydrate d'aluminium ou à la place de chlorhydrate d'aluminium est également présent un aluminium-zirconium-chlorhydrate.

9. Préparation pour la protection de la peau selon l'une quelconque des revendications 1 à 8, **caractérisée en ce que** ladite préparation comprend un composé en tant qu'agent humectant qui augmente l'hydratation de la peau d'environ 100 unités DPM à 300 - 600 unités DPM.

10. Préparation pour la protection de la peau selon l'une quelconque des revendications 1 à 10, **caractérisée en ce que** ladite préparation comprend en tant qu'agent humectant de la glycérine, du sorbitol, aloe vera, lactate de sodium et/ou urée, de préférence de la glycérine.

11. Préparation pour la protection de la peau selon l'une quelconque des revendications 1 à 10, **caractérisée en ce que** ladite préparation comprend en tant que lipide une huile végétale et/ou une graisse végétale telles qu'obtenues à partir de fleurs de tournesol, amandes, chardons, graines de soja, huile de shée, germes de blé, olives, cacahuètes, colza ou jojoba, et/ou des esters d'acide gras, de préférence isopropyl myristate ; (C₁₂-C₁₅)-alkyl-benzoate, 13-acide-docosénilique-9-octadecenylester (oléyl-erucate), cétéaryl-iso-nanoate, octyl palmitate, isooctyl stéarate, butyl adipate, myristyl lactate, stéaryl heptanoate, isostéaryl néopentanoate et/ou 2-octyldodécanol.

12. Préparation pour la protection de la peau selon l'une quelconque des revendications 1 à 11, **caractérisée en ce que** ladite préparation est présente sous une forme cosmétiquement acceptable, topiquement applicable, de préférence sous forme d'émulsion huile dans eau, d'émulsion eau dans huile, de solution aqueuse ou aqueuse/alcoolique, de lotio, gel, mousse, pommade ou pâte, ou sous une forme mélangée correspondante, de préférence sous forme d'émulsions eau dans huile, d'émulsion huile dans eau, de solution aqueuse/alcoolique ou de pommade, de préférence en émulsion huile dans eau sous forme d'une lotio, d'un gel ou d'une pommade.

13. Préparation pour la protection de la peau selon l'une quelconque des revendications 1 à 12, **caractérisée en ce que** ladite préparation contient un additif cosmétiquement acceptable choisi dans le groupe des agents émulsifiants, tensioactifs, hydrocarbures de paraffine, agents de consistance, gélifiants, agents de conservation, parfums, silicones, agents graissants, gaz propulseurs, agents de remplissage, colorants et/ou solvants.

14. Préparation pour la protection de la peau selon l'une quelconque des revendications 1 à 13, **caractérisée en ce que** la proportion en poids du constituant a) : constituant b) : constituant c) se situe dans une zone de 0.25-4 : 0.25-4 : 0.75-12, de préférence 0.5-2 : 0.5-2 : 1.5-6, particulièrement préféré 1 : 1 : 3.

15. Préparation pour la protection de la peau selon l'une quelconque des revendications 1 à 14, **caractérisée en ce que** ladite préparation contient environ 5 % en poids de chlorhydrate d'aluminium activé (de préférence REACH 301 ou REACH 501) en tant que constituant (a) ; environ 5 % en poids de glycérine en tant que constituant (b) ; et environ 8 % en poids d'huile de paraffine, environ 4 % en poids de palmitate octylique et environ 4 % en poids d'huile de jojoba en tant que constituant (c) ; environ 0.5 % en poids de diméthicone ; optionnellement de l'eau (env. 63 % en poids) ainsi que d'autres additifs qui sont nécessaires pour la fabrication d'une certaine forme d'utilisation.

16. Utilisation de la préparation selon l'une quelconque des revendications 1 à 15, en tant que moyen de protection de la peau.
